# EUROPEAN PATENT APPLICATION

(11) **EP 4 508 988 A1**
(43) Date of publication of application: **19.02.2025**
(21) Application number: 23020474.5
(22) Date of filing: 17.10.2023
(51) Int. Cl.: A23L 29/231, A23L 29/256, A23L 29/30, A23L 33/135, C12N 1/20

(54) **FERMENTED DIETARY PRODUCT, PROCESS FOR ITS PREPARATION AND USE**

(30) Priority: 18.08.2023 BG 11375423
(71) Applicant: DAFLORN Ltd., 9300 Dobrich (BG)
(72) Inventor: Peneva, Maria, 1618 Sofia (BG); Peneva, Daniela, 1618 Sofia (BG); Aleksandrov, Nikola, 1415 Sofia (BG); Aleksandov, Georgi, 1415 Sofia (BG)
(74) Representative: Stefanova, Stanislava Hristova

(57) **Abstract**

The invention relates to a fermented dietary product, process of its preparation based on plant raw materials and including selected strains of probiotic microorganisms specific for their characteristics, which has a marked health effect and is suitable as a food for people on a vegetarian or vegan diet as well as in accordance with religious concepts of nutrition. The fermented dietary product includes the plant raw materials pea protein, coconut flour, coconut milk, pectin and carrageenan and also strains Lactobacillus delbrueckii subsp. bulgaricus and Streptococcus thermophillus, isolated from sources of the territory of Bulgaria, more precisely:
Strain Lactobacillus delbrueckii subsp. bulgaricus DXB, registered in CCM under N_{º} 9196,
Strain Lactobacillus delbrueckii subsp. bulgaricus DXA, registered in CCM under N_{º} 9197,
Strain Lactobacillus delbrueckii subsp. bulgaricus DSW, registered in CCM under N_{º} 9198,
Strain Lactobacillus delbrueckii subsp. bulgaricus DLP, registered in CCM under N_{º} 9199,
Strain Lactobacillus delbrueckii subsp. bulgaricus DDM1, registered in CCM under N_{º} 9200 and
Strain Streptococcus thermophilus DWT4, registered in CCM under N_{º} 7992.

## Description

### Field of invention

The present invention relates to a fermented dietary product, process for its preparation and use as a dietary food or food supplement.

### Background

Increased food intolerance in the recent years, especially to lactose and gluten, popular dietary habits of avoiding foods of animal origin, called vegetarian or vegan, as well as the various religious concepts about certain types of nutrition, have led to an increased demand for processed foods guaranteeing the absence of unwanted ingredients and at the same time having good organoleptic qualities, which are as close as possible to the conventional food products. Therefore, many manufacturers have focused their efforts on obtaining fermented food products based on ingredients of plant origin. In most cases, however, these products have uncertain health effects, unpleasant aroma, low protein content, low content of live probiotic bacteria and poor texture, which affects their qualities as healthy foods.

For example, patent application WO2020128171 A1 claims a process for producing a fermented food product of vegetable origin that has organoleptic characteristics comparable to those of the traditional fermented dairy products obtained from milk of vegetable origin. As raw materials protein-oil plants are used in combination with soy milk and cashews in the form of whole nuts or pieces thereof or cashew milk, and fermentation is carried out with at least one of the following microorganisms: *Brevibacterium linens, Candida valida, Corynebacterium flavescens, Hafnia alvei, Kluyveromyces lactis, Lactobacillus acidophilus, Lactobacillus bulgaricus, Lactobacillus paracasei, Lactobacillus plantarum, Lactobacillus rhamnosus, Lactococcus cremoris, Lactococcus diacetylactis, Lactococcus lactis, Leuconostoc cremoris, Candida yeast, Staphylococcus carnosus, Staphylococcus Xylosus, and*/*or Streptococcus thermophiles,* as well as *Brevibacterium casei, Brevibacterium linens, Candida colliculose, Debaryomyces hanseii, Geotrichum candidum, Kluyveromyces marxianus, Pénicillium candidum, Pénicillium commune, Penicillium nalgiovensis, Penicillum Roqueforti, Trichosporon.*

However, the use of such a wide range of microorganism species, each of which includes multiple strains with their specific morphological and physiological characteristics, for the fermentation of an equally wide range of plant species could not lead to the unambiguous, beneficial for the consumers, qualities and properties of the product obtained by the process according to the invention. In addition, the wide range of the raw materials and microorganisms used does not allow conclusions to be drawn regarding the use of the product for healthy eating and safety in terms of allergens, antimicrobial properties, probiotic properties, maintenance of the normal functions of the body, etc.

The object of the invention is to provide a fermented dietary product and a process for its preparation, using vegetable raw materials and a set of specific strains of probiotic microorganisms, which to has a pronounced health effect and to be suitable for vegetarian or vegan diet, as well as to be compatible with the religious concepts of nutrition, for the purposes of healthy nutrition.

### Summary of the invention

The invention relates to a fermented dietary product based on plant raw materials and involving selected strains of probiotic microorganisms, to a process of preparation of the fermented dietary product and its use as food for people following vegetarian, vegan or parve diet, for effects such as improving lactose digestion in people with lactose intolerance, as well as for maintaining the normal functions of various organs and systems of the human body.

According to one aspect of the present invention, the fermented dietary product comprises the vegetable raw materials pea protein, coconut flour, coconut milk, pectin and carrageenan, as well as strains of *Lactobacillus delbrueckii* subsp. *bulgaricus* and *Streptococcus thermophillus,* isolated from sources on the territory of Bulgaria and selected from the group, consisting of:
Strain *Lactobacillus delbrueckii* subsp. *bulgaricus* DXB, registered in CCM under **N_{º}** 9196,
Strain *Lactobacillus delbrueckii* subsp. *bulgaricus* DXA, registered in CCM under **N_{º}** 9197,
Strain *Lactobacillus delbrueckii* subsp. *bulgaricus* DSW, registered in CCM under **N_{º}** 9198,
Strain *Lactobacillus delbrueckii* subsp. *bulgaricus* DLP, registered in CCM under **N_{º}** 9199,
Strain *Lactobacillus delbrueckii* subsp. *bulgaricus* DDM1, registered in CCM under **N_{º}** 9200 and
Strain *Streptococcus thermophilus* DWT4, registered in CCM under **N_{º}** 7992.

Species affiliation of the rods has been established through the ARDRA process (amplified ribosomal DNA restriction analysis) following *Giraffa et al.* where the strain *Lactobacillus delbrueckii* subsp. *bulgaricus* ATCC 11842 has been used as reference culture.

Differentiation of the individual rod strains was performed by macrorestriction analysis using pulse electrophoresis of the CHEF-DR II BioRad system. For the restriction analysis of the genomic DNA the enzyme X*hoI* was used for the rods. The results show a total of 5 genetic profiles corresponding to 5 new original strains of *Lactobacillus delbrueckii* subsp. *bulgaricus.*

Another aspect of the present invention relates to a process for preparation of a fermented dietary product based on the vegetable raw materials as stated above, subjected to a double fermentation with the participation of a selected combination of the above-mentioned strains of microorganisms and lyophilization.

Based on the knowledge that *Lactobacillus delbrueckii subsp. bulgaricus* should cooperate with *Streptococcus thermophilus* during lactic acid fermentation in the human and animal bodies and in order to carry out the fermentation according to the invention, the strain owned by the Applicant of the present invention *Streptococcus thermophilus* DWT4, registered in CCM under No. 7992 and a subject of Patent US9131708, was used.

In a further aspect, the present invention relates to a fermented dietary product which after lyophilization and after storage at a room temperature up to 24°C for 24 months contains live cells of the species *Lactobacillus delbrueckii* subsp. *bulgaricus* in amount of 2.8 × 10⁸ cfu/g to 5.0 × 10⁸ cfu/g, and of *Streptococcus thermophilus* species in amount of 1.7 × 10⁷ cfu/g to 5.4 × 10⁷ cfu/g.

Still another aspect of the present invention relates to the use of the fermented dietary product alone or in combination with plant extracts, trace elements and etc. as a dietary food, which is safe, including in regard to allergens, that has a proven bacteriostatic and bactericidal effect and each of the developed variants of the product exhibit efficiencies indicated for: maintenance of the normal function of the immune system; improvement of the lactose digestion in individuals with lactose intolerance; protection of the lipids in blood from oxidative stress; maintenance of the normal condition of bones and teeth; maintenance of the normal condition of hair, nails and skin; maintenance of the normal vision and function of the nervous system; protection of the cells from oxidative stress; normal carbohydrate metabolism, as well as to the normal protein and DNA synthesis.

### Brief description of the drawings

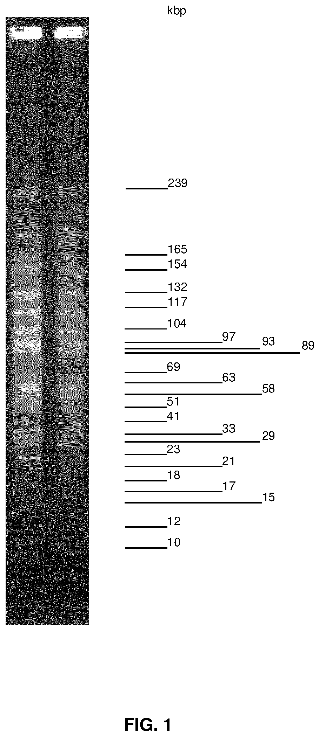

**Fig. 1** shows a macrorestriction profile of genomic DNA of an isolate of *Lactobacillus delbrueckii* subsp. *bulgaricus* corresponding to the strain *Lactobacillus delbrueckii* subsp. *bulgaricus* DSW, registered in CCM under No. 9198

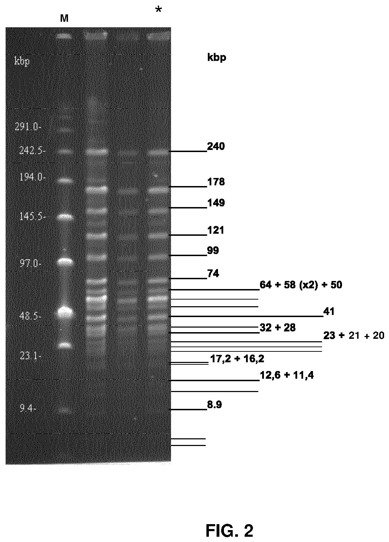

**Fig. 2** shows a macrorestriction profile of genomic DNA of an isolate of *Lactobacillus delbrueckii* subsp. *bulgaricus* corresponding to the strain *Lactobacillus delbrueckii* subsp. *bulgaricus* DXA, registered in CCM under No. 9197

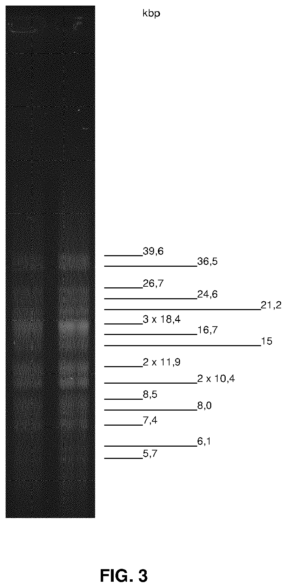

**Fig. 3** shows a macrorestriction profile of genomic DNA of an isolate of *Lactobacillus delbrueckii* subsp. *bulgaricus* corresponding to the strain *Lactobacillus delbrueckii* subsp. *bulgaricus* DDM1, registered in CCM under No. 9200

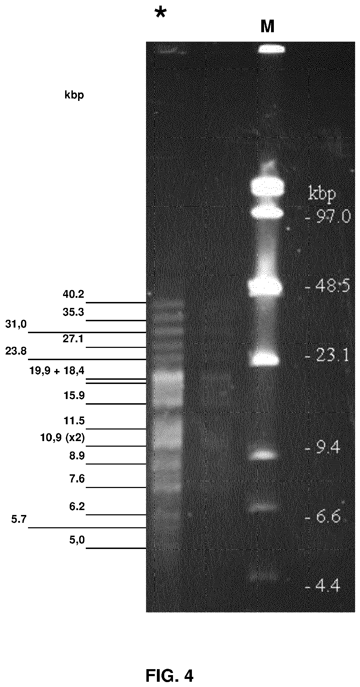

**Fig. 4** shows a macrorestriction profile of genomic DNA of an isolate of *Lactobacillus delbrueckii* subsp. *bulgaricus* corresponding to the strain *Lactobacillus delbrueckii* subsp. *bulgaricus* DXB, registered in CCM under No. 9196

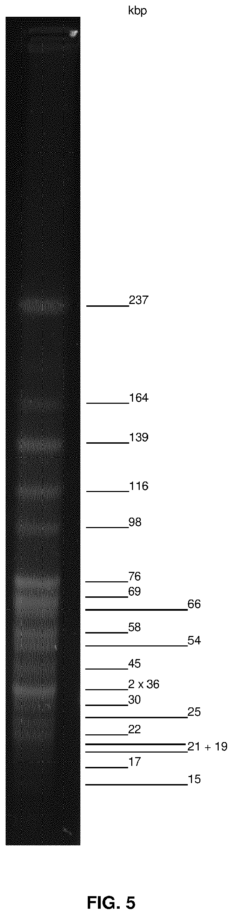

**Fig. 5** shows a macrorestriction profile of genomic DNA of an isolate of *Lactobacillus delbrueckii* subsp. *bulgaricus* corresponding to the strain *Lactobacillus delbrueckii* subsp. *bulgaricus* DLP, registered in CCM under No. 9199.

### DETAILED DESCRIPTION OF THE INVENTION

Preparation of the fermented dietary product, according to the invention, follows the technological sequence of operations, shown in Scheme 1 below:

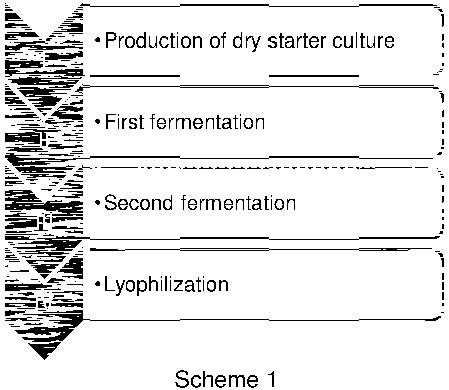

### Stage I- preparation of a dry starter culture; the input raw materials are expressed in wt% (weight percentages) relative to the total amount of the raw materials used in this stage.

A medium is prepared which include: dry pea protein - from 3.51 to 3.68; dry coconut flour- from 1.76 to 1.87; dry coconut milk- from 3.51 to 3.68; pectin- from 0.88 to 0.99; carrageenan- from 0.22 to 0.33; sucrose- from 0.44 to 0.55; dextrose monohydrate- from 0.44 to 0.55 and bottled spring water-from 79.03 to 87.82.

The mixture is homogenized, autoclaved at 121 °C for 5 min. followed by cooling to a temperature of 37°C to 45°C and fermented with 1.3 to 1.5 wt% starter culture, which includes a strain *Lactobacillus delbrueckii* subsp. *bulgaricus,* selected from the group consisting of the strains registered in the Czech Collection of Microorganisms (CCM) under the numbers 9196, 9197, 9198, 9199 and 9200 and a strain *Streptococcus thermophilus,* registered in the CCM under No. 7992 in a ratio of 1:1. It is put in a thermostat at a temperature of 37°C to 45°C to reach a pH of 4,1 to 4,5. A pH and a swab samples are collected. Microorganisms are stained in methylene blue suitable for Gram (+) bacteria. The microscopic picture shows the presence of short rods and cocci with morphology specific to *Lactobacillus delbrueckii* subsp. *bulgaricus* and *Streptococcus thermophilus.* The liquid starter culture obtained after fermentation is cooled down to 20°C.

A 50% sugar syrup is prepared with bottled spring water in a ratio of 1:1 by heat treatment at 100°C for 30 min. The syrup is cooled down to 20°C, then added to the liquid starter culture as a cryoprotectant. The mixture is homogenized and lyophilized. The resulting dry starter culture is stored at a temperature of -18° to -22° C.

### Stage II - Preparation of a liquid fermented product 1; the input raw materials are expressed in wt%.

With continuous stirring, a medium is prepared consisting of: dry pea protein - from 1.02 to 1.22; dry coconut milk - from 1.02 to 1.22; dry coconut flour - from 0.51 to 0.61; pectin - from 0.25 to 0.26; carrageenan - from 0.06 to 0.07; sucrose - from 0.12 to 0.14; dextrose monohydrate - from 0.12 to 0.14 and bottled spring water - from 22.34 to 23.97. The mixture is sterilized at 98°C for 60 min. It is cooled down to 37°- 45°C. The measured pH of the medium is from 6.1 to 6.3.

The medium thus prepared is inoculated with the dry starter culture obtained **in Stage I** in an amount from 0.08 to 0.1 and left to ferment for 16-18 hours until a pH of 4.1 to 4.5 is reached. A pH and a swab samples are collected. Microorganisms are stained in methylene blue suitable for Gram (+) bacteria. The microscopic picture shows the presence of short rods and cocci with morphology specific to *Lactobacillus delbrueckii subsp. bulgaricus* and *Streptococcus thermophilus.* The resulting product is *liquid fermented product 1.*

### Stage III - Preparation of the liquid fermented product 2

With continuous stirring, a medium is prepared consisting of: dry pea protein - from 2.64 to 2.72; dry coconut milk - from 2.64 to 2.72; dry coconut flour - from 1.32 to 1.36; pectin - from 0.66 to 0.67; carrageenan - from 0.16 to 0.17; sucrose - from 0.33 to 0.34; dextrose monohydrate - from 0.33 to 0.34 and bottled spring water - from 61.75 to 62.93. The mixture is sterilized at 98°C for 60 min. and cooled down to 37°C to 45°C. The measured pH of the medium is from 6.1 to 6.3.

A cryoprotectant is prepared from sugar, dextrose monohydrate and bottled spring water in a ratio of 1.5:1.2:2, then subjected to heat treatment up to 100°C for 30 min., cooled down to a temperature of 37°- 45°C and then added to the liquid medium obtained in this stage. The liquid fermented product 1 from Stage 2 is added to the resulting mixture in a ratio of 1:3, then homogenized and left to ferment again for 1 to 3 hours at a temperature of 37°- 45°C until pH of 4.5 to 4.7 is reached. When the desired pH is reached, a swab is taken. Microorganisms are stained in methylene blue suitable for Gram (+) bacteria. The microscopic picture shows the presence of short rods and cocci with morphology specific to *Lactobacillus delbrueckii* subsp. *bulgaricus* and *Streptococcus thermophilus.* The resulting product is *liquid fermented product 2,* which is cooled down to 20°C.

**Stage IV-** This stage of the technological scheme includes lyophilization of the liquid fermented product 2. The resulting final lyophilized dietary product has a dry substance from 12.1 to 13.31 wt%, including all raw materials input in stages I, II, III and IV of the technological scheme. The resulting dry fermented dietary product has a powdery to finely flaky consistency; light beige color; specific sweet-sour taste and slight coconut smell. It contains a maximum of 6% moisture and after rehydration it has a pH of 5,5 to 6,9. It is available in dry form or in capsules.

It is stored at a temperature of 20 to 24°C and humidity of up to 60%.

The process of producing a fermented dietary product, according to the present invention, allows both the use of each of the strains of microorganisms separately, dictated by their specific physiological features for obtaining products specific in terms of their applicability and also, adding various additives to the end product - a dry fermented dietary product, such as dry extract of olive fruits (carrier of polyphenols including hydroxytyrosol), magnesium oxide (carrier of active magnesium), zinc pidolate (carrier of active zinc), inulin (prebiotic) and etc. with a view to meeting the specific needs of the consumers.

All variants of the product, obtained by the process according to the invention, are illustrated below with corresponding exemplary embodiments.

The species composition and the numbers of the individual groups of microorganisms, contained in the product variants at the date of manufacture, according to the invention, are presented in Table 1. The results of the number of the grown colonies on M17 and MRS agar show that all rod isolates belong to the species *Lactobacillus delbrueckii* subsp. *bulgaricus* in an amount from 2.9×10⁸ to 5.2×10⁸ CFU/g, and all cocci isolates belong to the species *Streptococcus thermophilus* and their amount is from 1.8×10⁷ to 5.6×10⁷ CFU/g. The higher number of the rods as compared to the number of cocci is noticeable.

**Table 1**

| Product/ combination of microorganisms | Lactobacillus delbrueckii subsp. bulgaricus (CFU/g) | Streptococcus thermophilus (CFU/g) |
|---|---|---|
| Product 1, fermented with *Lactobacillus delbrueckii subsp. bulgaricus* DXB, CCM Nº 9196 and *Streptococcus thermophilus* DWT4 CCM Nº 7992 | 3,3 x 10⁸ - 4,2 x 10⁸ | 4,2 x 10⁷ - 5,4 x 10⁷ |
| Product 2, fermented with *Lactobacillus delbrueckii subsp. bulgaricus* DXA, CCM Nº 9197 and *Streptococcus thermophilus* DWT4, CCM Nº 7992 | 3,5 x 10⁸ - 4,6 x 10⁸ | 4,9 x 10⁷ - 5,6 x 10⁷ |
| Product 3, fermented with *Lactobacillus delbrueckii subsp. bulgaricus* DSW, CCM Nº 9198 and *Streptococcus thermophilus* DWT4, CCM Nº 7992 | 4,0 x 10⁸ - 4,9 x 10⁸ | 3,5 x 10⁷ - 4,3 x 10⁷ |
| Product 4, fermented with *Lactobacillus delbrueckii subsp. bulgaricus* DLP, CCM Nº 9199 and *Streptococcus thermophilus* DWT4, CCM Nº 7992 | 2,9 x 10⁸ - 3,6 x 10⁸ | 1,8 x 10⁷ - 2,7 x 10⁷ |
| Product 5, fermented with *Lactobacillus delbrueckii subsp. bulgaricus* DDM1, CCM Nº 9200 and *Streptococcus thermophilus* DWT4, CCM N_{º} 7992 | 4,2 x 10⁸ - 5,2 x 10⁸ | 4,8 x 10⁷ - 5,6 x 10⁷ |

The species composition and the numbers of the individual groups of microorganisms, contained in the product variants according to the invention, after 24 months of storage at a temperature of up to 24°C are presented in Table 2. The results of the grown colonies on M17 and MRS agar show that all rod isolates belong to the species *Lactobacillus delbrueckii* subsp. *bulgaricus* in an amount from 2.8×10⁸ to 5.0×10⁸ CFU/g, and all the cocci isolates belong to *Streptococcus thermophilus* species in an amount from 1.7×10⁷ to 5.4×10⁷ CFU/g. The trend for a higher rod counts is maintained. The results show that the number of the individual groups of microorganisms, contained in the product variants, is preserved with minor changes after 24 months of storage at a temperature of 24°C.

**Table 2**

| Product/ combination of microorganisms | Lactobacillus delbrueckii subsp. bulgaricus (CFU/g) | Streptococcus thermophilus (CFU/g) |
|---|---|---|
| Product 1, fermented with *Lactobacillus delbrueckii subsp. bulgaricus* DXB, CCM Nº 9196 and *Streptococcus thermophilus* DWT4 CCM Nº 7992 | 3,1 x 10⁸ - 4,1 x 10⁸ | 4,0 x 10⁷ - 5,2 x 10⁷ |
| Product 2, fermented with *Lactobacillus delbrueckii subsp. bulgaricus* DXA, CCM Nº 9197 and *Streptococcus thermophilus* DWT4, CCM Nº 7992 | 3,3 x 10⁸ - 4,5 x 10⁸ | 4,8 x 10⁷ - 5,4 x 10⁷ |
| Product 3, fermented with *Lactobacillus delbrueckii subsp. bulgaricus* DSW, CCM Nº 9198 and *Streptococcus thermophilus* DWT4, CCM Nº 7992 | 3,8 x 10⁸ - 4,8 x 10⁸ | 3,4 x 10⁷ - 4,1 x 10⁷ CFU |
| Product 4, fermented with *Lactobacillus delbrueckii subsp. bulgaricus* DLP, CCM Nº 9199 and *Streptococcus thermophilus* DWT4, CCM Nº 7992 | 2,8 x 10⁸ - 3,5 x 10⁸ | 1,7 x 10⁷ - 2,5 x 10⁷ |
| Product 5, fermented with *Lactobacillus delbrueckii subsp. bulgaricus* DDM1, CCM Nº 9200 and *Streptococcus thermophilus* DWT4, CCM Nº 7992 | 4,0 × 10⁸ - 5,0 × 10⁸ | 4,6 × 10⁷ - 5,4 × 10⁷ |

The fermented dietary product in its variants according to the invention have been tested for safety as per the requirements of HACCP (Hazard Analysis and Critical Control Point). The results of the study of product 1, containing *Lactobacillus delbrueckii* subsp. *bulgaricus* DXB, CCM N_{º} 9196 and *Streptococcus thermophilus* DWT4, CCM N_{º} 7992, are presented in Table 3. The results indicate that the product is safe for human consumption. The results of the study of products 2, 3, 4 and 5 are similar and indicate that they are also safe for human consumption.

**Table 3**

| INDICATORS | ANALYSIS PROCESSS | CHARACTERISTICS AND NORMS | RESULTS |
|---|---|---|---|
| Heavy metals and other toxic elements, mg/kg, no more than: | Arsenic under BDS EN 14546:2006 Lead, cadmium and copper under BDS EN 14084:2003 Mercury under BDS EN 13806:2003 | Copper-3,0; lead- 1,0; arsenic-2,0; cadmium-0,1; mercury-0,04; | Complies |
| Total number of mesophilic aerobic and facultative anaerobic non-lactic acid bacteria, CFU/g, no more than: | BDS ISO 6610 | 1000 (1,0. 10³) | Complies |
| Coliforms in 1.0 g of the product: | BDS ISO 4831 | Not to be established | Complies |
| Yeast and mold in 1,0 g of the product: | BDS ISO 6611 | Not to be established | Complies |
| Microscopic mold fungi spores in 1.0 g of the product: | BDS ISO 6611 | Not to be established | Complies |
| *Salmonella species* in 25,0 g of the product: | BDS ISO 6579 | Not to be established | Complies |
| Coagulasopositive *staphylococci* (*S. aureus*) in 1,0 g of the product: | BDS ISO 6888-1,2,3 | Not to be established | Complies |
| *Listeria monocytogenes* in 25,0 g of the product: | BDS ISO 11290 - 1 | Not to be established | Complies |
| Aflatoxin M1 mg/kg | BDS ISO 16242 - 85 | No more than 0.5 | Complies |

In addition, the fermented dietary product, according to the invention, also meets the safety requirements regarding allergens according to Regulation (EU) N_{º} 1169/2011, Annex II of the European Parliament and Council and it does not contain allergens (1).

Antimicrobial effect: Antimicrobial activity of the strains of *Lactobacillus delbrueckii* subsp*. bulgaricus,* involved in the fermented dietary product, according to the present invention, has been proven by establishing the bacteriostatic and bactericidal effect of the dry product samples by means of well diffusion process. Studies of the relationships between the lyophilized product and pathogenic microorganisms, reference cultures, clinical isolates (intestinal, faecal) and those isolated from food pathogens of the following species: *Escherichia coli* serotypes O6, O44 and O127 and *Shigella flexneri* have been done. For this purpose pathogenic microorganisms are grown on *Nublent agar* (MERK, 1.005450.0500) with added 0,5% bile salts at a concentration of 10⁵ CFU/ml, and the product is previously rehydrated by sterile distilled water in a ratio of 1:3. Capacity of the wells in which the product is dripped is 50pl. The cultures were incubated for 24-48 h at 37°C in an anaerobic medium (ANAEROGEN 0035A, OXOID). The interaction was reported as bacteriostatic (BS) and bactericidal (BC) in millimeters of the inhibition zone or sterile zone. The results of the experimental study against pathogenic microorganisms are summarized in Table 4 below.

**Table 4**

| Test microorganisms | | Products | | | | |
|---|---|---|---|---|---|---|
| | | Product 2 - LBB DXA, CCM 9197 | Product 1 - LBB DXB, CCM 9196 | Product 5 - LBB DDM1, CCM 9200 | Product 4 - LBB DLP, CCM 9199 | Product 3 - LBB DSW, CCM 9198 |
| *Escherichia coli* | BC (mm) | 0 | 0 | 1 | 0 | 2 |
| | BS (mm) | 5 | 4 | 5 | 4 | 5 |
| *Shigella flexneri* | BC (mm) | 2 | 1 | 2 | 0 | 1 |
| | BS (mm) | 5 | 4 | 3 | 3 | 4 |

The results show that the products containing the strains of *Lactobacillus delbrueckii* subsp. *bulgaricus,* according to the invention, exhibit good antimicrobial activity, including a remarkable bacteriostatic effect and a moderate bactericidal effect. The bacteriostatic and bactericidal effects are most pronounced in the product containing *Lactobacillus delbrueckii* subsp. *bulgaricus* DSW, CCM N_{º} 9198 and *Streptococcus thermophilus* DWT4, CCM N_{º} 7992.

The invention is illustrated by the following Examples without limitation of its scope:

### Example 1: Depending on the type of the microorganisms used in the process, according to the invention, the following products are obtained:

Product 1- Following the technological scheme, the first step is to obtain dry starter culture (Stage I). For this purpose, a medium containing 66.0 g dry pea protein, 34 g dry coconut flour, 67 g dry coconut milk, 18 g pectin, 6 g carrageenan, 9 g sucrose, 9 g dextrose and 1440 ml bottled spring water is prepared. The mixture is homogenized and autoclaved at 121 °C for 5 min. and then cooled down to a temperature of 37°C to 45°C. It is fermented with 12 g of the strain *Lactobacillus delbrueckii* subsp. *bulgaricus* DXB, CCM N_{º} 9196, isolated from the udder of a domestic cow in the Rhodope region and 12 g of the strain *Streptococcus thermophilus* DWT4, CCM N_{º} 7992 of water origin, then it is placed in a thermostat at a temperature of 37°C to 45°C for 16-18 hours until pH of 4.1 to 4.3 is reached. A sample for pH and swab are collected. Microorganisms are stained in methylene blue suitable for Gram (+) bacteria. The microscopic picture shows the presence of short rods and cocci with morphology specific to *Lactobacillus delbrueckii* subsp. *bulgaricus* and *Streptococcus thermophilus.* The resulting liquid starter culture is cooled down to 20°C.

170 ml of 50% sugar syrup is prepared from bottled spring water and sucrose in a ratio of 1:1 by heat treatment at 100°C for 30 min. The syrup is cooled down to 20°C, then added to the liquid starter culture as a cryoprotectant. The mixture is homogenized and lyophilized. The resulting dry starter culture is stored at -18°C.

Stage II includes the preparation of a liquid fermented product 1. A medium is prepared from 3 kg dry pea protein, 2.5 kg dry coconut milk, 1.25 kg dry coconut flour, 0.640 kg pectin, 0.165 kg carrageenan, 0.3 kg sucrose, 0.3 kg dextrose monohydrate and 59 l bottled spring water. The mixture is sterilized at 98°C for 60 min. It is cooled down to a temperature of 37°C to 45°C. The measured pH of the medium is from 6.1 to 6.3. The medium thus prepared is inoculated with 200 g dry starter culture, obtained in Stage 1 and then it is fermented for 16-18 hours until pH of 4.1 to 4.3 is reached. A pH sample and swab are collected. Microorganisms are stained in methylene blue suitable for Gram (+) bacteria. The microscopic picture shows the presence of short rods and cocci with morphology specific to *Lactobacillus delbrueckii* subsp. *bulgaricus* and *Streptococcus thermophilus.*

Stage III includes the preparation of a liquid fermented product 2 (LFP2). With continuous stirring a medium is prepared consisting of 6.7 kg dry pea protein, 6.5 kg dry coconut milk, 3.35 kg dry coconut flour, 1.640 kg pectin, 0.4 kg carrageenan, 0.825 kg sucrose, 0.825 kg dextrose monohydrate and 152 I bottled spring water. The mixture is sterilized at 98°C for 60 min. Then it is cooled down to a temperature of 37°C to 45°C. The measured pH of the medium is from 6.1 to 6.3.

12 l cryoprotectant is prepared consisting of sugar, dextrose monohydrate and bottled spring water in a ratio of 1.5:1.2:2 by heat treatment up to 100°C for 30 min. It is cooled down to a temperature of 37°C to 45°C. Thus prepared the cryoprotectant is added to the liquid medium obtained at this stage. The liquid fermented product 1 from Stage 2 is added to the obtained mixture, then it is homogenized and fermented again for 1 to 3 hours at a temperature of 37°C to 45°C until pH from 4.5 to 4.7 is reached. A pH sample and swab are collected. Microorganisms are stained in methylene blue suitable for Gram (+) bacteria. The microscopic picture shows the presence of short rods and cocci with morphology specific to *Lactobacillus delbrueckii* subsp. *bulgaricus* and *Streptococcus thermophilus.* The resulting liquid fermented product 2 is cooled down to 20°C and then lyophilized.

The resulting dry lyophilized product has a weight of 30.7 kg. The product is stored at a temperature of 20°C - 24°C and humidity up to 60%, as after storage for 24 months it contains live cells of the species *Lactobacillus delbrueckii* subsp. *bulgaricus* in an amount of 3.1 × 10⁸ to 4.1 × 10⁸ CFU/g and of the species *Streptococcus thermophilus* in an amount of 4.0 × 10⁷ to 5.2 × 10⁷ CFU/g.

The daily dose for human intake is a minimum of 1 g in 24 hours. The product is dissolved in water, tea, juice or any other liquid with a temperature up to 37°C to keep microorganisms alive. It is taken in the morning on an empty stomach 20 minutes before meal.

Product 2 - This product is produced according to the same scheme as for Product 1, the difference is that the strain *Lactobacillus delbrueckii* subsp. *bulgaricus* DXA, CCM N_{º} 9197, isolated from the udder of a domestic cow in the Rhodopes region and the strain *Streptococcus thermophilus* DWT4, CCM N_{º} 7992 of water origin are used as active microorganisms. The resulting dry fermented dietary Product 2 is stored at a temperature of 20-24°C and humidity up to 60%, as after storage for 24 months it contains live cells of the species *Lactobacillus delbrueckii* subsp. *bulgaricus* in an amount of 3.3 × 10⁸ to 4.5 × 10⁸ CFU/g and of the species *Streptococcus thermophilus* in an amount from 4.8 × 10⁷ to 5.4 × 10⁷ CFU/g. The daily intake dose is a minimum of 1 g in 24 hours.

Product 3- The product is obtained according to the same scheme as for products 1 and 2, the difference is that the strain *Lactobacillus delbrueckii* subsp*. bulgaricus* DSW, CCM N_{º} 9198, isolated from mountain spring water in Osogovska planina, and the strain *Streptococcus thermophilus* DWT4, CCM N_{º} 7992 of water origin are used as active microorganisms. The resulting dry fermented dietary Product 3 is stored at a temperature of 20-24°C and humidity up to 60%, as after storage for 24 months it contains live cells of the species *Lactobacillus delbrueckii* subsp. *bulgaricus* in an amount of 3.8 × 10⁸ to 4.8 × 10⁸ CFU/g and of the species *Streptococcus thermophilus* in an amount of 3,4 × 10⁷ to 4,1 × 10⁷ CFU/g. The daily intake dose is a minimum of 1 g in 24 hours.

Product 4- The product is obtained according to the same scheme as for products 1, 2 and 3, the difference is that the strain *Lactobacillus delbrueckii* subsp. *bulgaricus* DLP, CCM N_{º} 9199, isolated from faeces of free-range chickens in Dobrudja region and the strain *Streptococcus thermophilus* DWT4, CCM N_{º} 7992 of water origin are used as active microorganisms. The resulting dry fermented dietary Product 4 is stored at a temperature of 20-24°C and humidity up to 60%, as after storage for 24 months it contains live cells of the species *Lactobacillus delbrueckii* subsp. *bulgaricus* in an amount of 2.8 × 10⁸ to 3.5 × 10⁸ CFU/g and of the species *Streptococcus thermophilus* in an amount of 1.7 × 10⁷ to 2.5 × 10⁷ CFU/g. The daily dose for humans is a minimum of 1 g in 24 hours.

Product 5- The product is obtained according to the same scheme as for products 1, 2 and 3, the difference is that the strain *Lactobacillus delbrueckii* subsp. *bulgaricus* DDM1, CCM N_{º} 9200, isolated from bee honey in the Dobrudja region and the strain *Streptococcus thermophilus* DWT4, CCM N_{º} 7992 of water origin are used as active microorganisms. The resulting dry fermented dietary Product 5 is stored at a temperature of 20-24°C and humidity up to 60%, as after storage for 24 months it contains live cells of the species *Lactobacillus delbrueckii* subsp. *bulgaricus* in an amount of 4.0 × 10⁸ to 5.0 × 10⁸ CFU/g and of the species *Streptococcus thermophilus* in an amount of 4.6 × 10⁷ to 5.4 × 10⁷ CFU/g. The daily dose for humans is a minimum of 1 g in 24 hours.

### Example 2. Fermented dietary product for the protection of blood lipids from oxidative stress.

The product is obtained by mixing mechanically the dry fermented dietary product 2 according to Example 1 above and dry extract of olive fruit (*Olea Europea L.*) Oli Ola, standardized for polyphenols and hydroxytyrosol and then encapsulation. Content in 1 capsule (340 mg): 200 mg dry fermented dietary product 2, containing strain *Lactobacillus delbrueckii* subsp. *bulgaricus* DXA, CCM N_{º} 9197 and strain *Streptococcus thermophilus* DWT4, CCM N_{º} 7992; 85 mg dry extract of olive fruit, incl. 22.0 mg polyphenols and 2.5 mg hydroxytyrosol; 15 mg inulin (prebiotic) and 40 mg magnesium stearate. It is stored at a temperature of 20-24°C and humidity up to 60%. Daily dose for human consumption - 2 capsules taken in the morning with some liquid 20 minutes before meal.

The product complies with COMMISSION REGULATION (EU) No 432/2012 of 16 May 2012 (2) establishing a list of permitted health claims made on foods, other than those referring to the reduction of disease risk and to children's development and health, according to which polyphenols in olive oil contribute to the protection of blood lipids from oxidative stress if they contain a daily dose of 5 mg hydroxytyrosol. The product according to the invention contains 5 mg hydroxytyrosol divided into 2 capsules per day. The health claim of this product is consistent with the EFSA statement on the health claim for olive fruit extract (3).

### Example 3: Fermented dietary product with active magnesium.

The product is obtained by mixing mechanically the dry fermented dietary product 4 from Example 1 and magnesium oxide, containing active magnesium extracted from sea water (30%), and encapsulation. Content in 1 capsule (350 mg): 200 mg dry fermented dietary product 4 containing *Lactobacillus delbrueckii* subsp. *bulgaricus* DLP, CCM N_{º} 9199 and *Streptococcus thermophilus* DWT4, CCM N_{º} 7992; 103 mg magnesium oxide (Simag) incl. 57 mg active magnesium extracted from sea water (30%); 30 mg inulin (prebiotic) and 17 mg anti-hardener magnesium stearate. It is stored at a temperature of 20-24°C and humidity up to 60%. The daily dose for human consumption is 2 capsules taken in the morning with some liquid 20 minutes before meal. According to the COMMISSION REGULATION (EU) No 432/2012 of 16 May 2012 establishing a list of permitted health claims made on foods, other than those referring to the reduction of disease risk and to children's development and health, magnesium has 10 recognized health claims if the daily dose is 114 mg of active magnesium. The product contains 114 mg of active magnesium divided into 2 capsules per day. Some of the health claims of magnesium are that magnesium contributes to: a reduction of tiredness and fatigue; the normal functioning of the nervous system; normal muscle function; the maintenance of normal bones and teeth; normal protein synthesis and normal psychological function. The health claim of this product is consistent with the EFSA statement on the health claim for magnesium (4, 5).

### Example 4: Fermented dietary product with active zinc

The product is obtained by mixing mechanically the dry fermented dietary product 5 from Example 1 and zinc pidolate (Pido-Zinc) - carrier of active zinc and encapsulation. Content in 1 capsule (320 mg): 230.0 mg dry fermented dietary product 5 containing *Lactobacillus delbrueckii* subsp*. bulgaricus* DDM1, CCM N_{º} 9200 and *Streptococcus thermophilus* DWT4, CCM N_{º} 7992; 37.0 mg Pido-Zinc (zinc pidolate - carrier of 7,5 mg active zinc); 33.0 mg inulin (prebiotic) and 20.0 mg anti-hardener magnesium stearate (4). It is stored at a temperature of 20-24°C and humidity up to 60%. The daily dose for human consumption is 2 capsules taken in the morning with some liquid 20 minutes before meal. According to COMMISSION REGULATION (EU) No 432/2012 of 16 May 2012, zinc has 18 recognized health claims if the daily dose is 15 mg active zinc. The product according to the invention contains 15 mg of active zinc, divided into 2 capsules per day.

Some of the health claims of zinc are that zinc contributes to: the normal function of the immune system; normal carbohydrate metabolism; normal DNA synthesis; normal protein synthesis; normal cognitive function; normal fertility and reproduction; the maintenance of normal hair, nails, bones and skin and the protection of cells from oxidative stress (6).

### Example 5: Fermented dietary product for improving lactose digestion in individuals with lactose intolerance

The product is obtained by mechanically mixing the dry fermented dietary products 1, 2, 3, 4 and 5 of Example 1 in a ratio of 1:1:1:1:1 and homogenizing. It is stored at a temperature of 20°C to 24°C and humidity up to 60%. The daily intake dose for humans is a minimum of 10 g in 24 hours. The product is dissolved in water, tea, juice or other liquid with a temperature of up to 37°C to keep the microorganisms alive. It is taken in the morning on an empty stomach 20 minutes before meal.

According to COMMISSION REGULATION (EU) No 432/2012 of 16 May 2012, the probiotic microorganisms *Lactobacillus bulgaricus* and *Streptococcus thermophilus* improve lactose digestion in individuals who have difficulty digesting lactose when administered at a dose of 10⁸ to 10⁹ cfu/g. As can be seen from the data summarized in Table 1, the product in accordance with the present invention contains live cells of *Lactobacillus delbrueckii* subsp. *bulgaricus* species in an amount of 2.9 × 10⁸ cfu/g to 5.2 × 10⁸ cfu/g, and those of the *Streptococcus thermophilus* species are in an amount of 1.8 × 10⁷ cfu/g to 5.6 × 10⁷ cfu/g, which exceeds the recommended doses under the Regulation. The health claim of this product is consistent with the EFSA statement on the health claim of *Lactobacillus delbrueckii* subsp. *bulgaricus* and *Streptococcus thermophilus* (7)*.*

All the products described in detail in Examples 1 to 5 above are suitable both for maintaining the normal functions of various organs and systems of the human body and as a food for people following vegetarian, vegan or parve diet.

### REFERENCE

*1. Regulation (EU) No. 1169*/*2011 of the European Parliament and of the Council of 25 October 2011 on the provision of food information to consumers;* *http.*//*data.europa.eu*/*eli*/*reg*/*2011*/*1169*/*oj*
*2. Commission Regulation (EU) No. 432*/*2012 of 16 May 2012 establishing a list of permitted health claims made on foods, other than those referring to the reduction of disease risk and to children's development and health Text with EEA relevance,* *http:*//*data.europa.eu*/*eli*/*reg*/*2012*/*432*/*oj*
*3. Scientific Opinion on the substantiation of health claims related to polyphenols in olive and protection of LDL particles from oxidative damage (ID 1333, 1638, 1639, 1696, 2865), maintenance of normal blood HDL-cholesterol concentrations (ID 1639), maintenance of normal blood pressure (ID 3781), "anti-inflammatory properties" (ID 1882), "contributes to the upper respiratory tract health" (ID 3468), "can help to maintain a normal function of gastrointestinal tract" (3779), and "contributes to body defences against external agents" (ID* 3467*) pursuant to Article 13(1) of Regulation (EC) No 1924*/*2006,* EFSA Journal 2011;9(4):2033
*4. Scientific Opinion on the substantiation of health claims related to magnesium and electrolyte balance (ID 238), energy-yielding metabolism (ID 240, 247, 248), neurotransmission and muscle contraction including heart muscle (ID 241, 242), cell division (ID 365), maintenance of bone (ID* 239*)*, *maintenance of teeth (ID* 239*)*, *blood coagulation (ID 357) and protein synthesis (ID 364) pursuant to Article 13(1) of Regulation (EC) No 1924*/*20061,* EFSA Journal 2009; 7(9):1216
*5. Scientific Opinion on the substantiation of health claims related to magnesium and "hormonal health" (ID* 243*)*, *reduction of tiredness and fatigue (ID 244), contribution to normal psychological functions (ID 245, 246), maintenance of normal blood glucose concentrations (ID 342), maintenance of normal blood pressure (ID 344, 366, 379), protection of DNA, proteins and lipids from oxidative damage (ID 351), maintenance of the normal function of the immune system (ID 352), maintenance of normal blood pressure during pregnancy (ID 367), resistance to mental stress (ID 375, 381), reduction of gastric acid levels (ID 376), maintenance of normal fat metabolism (ID* 378*) and maintenance of normal muscle contraction (ID 380, ID 3083) pursuant to Article 13(1) of Regulation (EC) No 1924*/*2006,* EFSA Journal 2010;8(10):1807
*6. PIDO-ZINC, SPECIFICATIONS DATA SHEET, Produced & Commercialised by UCIB* - *SOLABIA Group, 26*/*04*/*2018*
*7. Scientific Opinion on the substantiation of health claims related to live yoghurt cultures and improved lactose digestion (ID 1143, 2976) pursuant to Article 13(1) of Regulation (EC) No 1924*/*20061,* EFSA Journal 2010;8(10):1763

## Claims

1. Process of preparation of a fermented dietary product based on vegetable raw materials, **characterized in that** the vegetable raw materials, including pea protein, coconut flour, coconut milk, pectin and carrageenan, are subjected to a double fermentation involving strains of *Lactobacillus delbrueckii* subsp. *bulgaricus* and *Streptococcus thermophillus,* selected from the group including:
Strain *Lactobacillus delbrueckii subsp. bulgaricus* DXB, registered in CCM under No. 9196,
Strain *Lactobacillus delbrueckii subsp. bulgaricus* DXA, registered in CCM under N_{º} 9197,
Strain *Lactobacillus delbrueckii subsp. bulgaricus* DSW, registered in CCM under N_{º} 9198,
Strain *Lactobacillus delbrueckii subsp. bulgaricus* DLP, registered in CCM under N_{º} 9199,
Strain *Lactobacillus delbrueckii subsp. bulgaricus* DDM1, registered in CCM under N_{º} 9200 and
Strain *Streptococcus thermophilus* DWT4, registered in CCM under N_{º} 7992.

2. Process of preparation of a fermented dietary product according to claim 1, **characterized in that** the double fermentation is carried out in a technological sequence of operations in four stages as follows:
- Preparation of a dry starter culture;
- First fermentation to obtain a liquid fermented product 1 ;
- Second fermentation to obtain a liquid fermented product 2;
- Lyophilization

3. Process of preparation of a fermented dietary product according to claims 1 and 2, **characterized in that**, for preparation of the dry starter culture, a medium comprising of dry pea protein, dry coconut flour, dry coconut milk, pectin, carrageenan, sucrose, dextrose monohydrate and bottled spring water is prepared, which is homogenized and autoclaved at 121 °C for 5 min., then cooled down to a temperature of 37°C to 45°C and fermented with starter culture including strain *Lactobacillus delbrueckii* subsp. *bulgaricus,* selected from the group consisting of the strains deposited in CCM under Numbers 9196, 9197, 9198, 9199 and 9200 and strain *Streptococcus thermophilus,* deposited in CCM under No. 7992 in a 1:1 ratio, then it is put into a thermostat at a temperature of 37°C to 45°C to reach pH 4.1 - 4.5 and cooled down to 20°C, after which a cryoprotectant of 50% sugar syrup from bottled spring water and sucrose in 1:1 ratio is added with subsequent homogenization and lyophilization.

4. Process of preparation of a fermented dietary product according to claim 3, **characterized in that** the components involved in the preparation of the medium and the starter culture for its fermentation are in quantities as follows (wt %):
dry pea protein - from 3.51 to 3.68; dry coconut flour - from 1.76 to 1.87; dry coconut milk - from 3.51 to 3.68; pectin - from 0.88 to 0.99; carrageenan - from 0.22 to 0.33; sucrose - from 0.44 to 0.55; dextrose monohydrate - from 0.44 to 0.55 and bottled spring water - from 79.03 to 87.82; starter culture - from 1.3 to 1.5.

5. Process of preparation of a fermented dietary product according to claims 1-4, **characterized in that**, that for carrying out the first fermentation of preparation of a liquid fermented product 1, a medium comprising of a dry pea protein, dry coconut milk, dry coconut flour, pectin, carrageenan, sucrose, dextrose monohydrate and bottled spring water is prepared, which is then sterilized at 98°C for 60 min., cooled down to 37°- 45°C, inoculated with the dry starter culture according to claims 3 and 4 and then it is left to ferment for 16-18 hours until pH of 4.1 to 4.5 is reached.

6. Process of preparation of a fermented dietary product according 5, **characterized in that** the components involved in the preparation of the medium and the dry starter culture for obtaining the liquid fermented product 1 are in quantities as follows (in wt%):
dry pea protein - from 1.02 to 1.22; dry coconut milk - from 1.02 to 1.22; dry coconut flour - from 0.51 to 0.61; pectin - from 0.25 to 0.26; carrageenan - from 0.06 to 0.07; sucrose - from 0.12 to 0.14; dextrose monohydrate - from 0.12 to 0.14 and bottled spring water - from 22.34 to 23.97; dry starter culture - from 0.08 to 0.1.

7. Process of preparation of a fermented dietary product according to claims 1-6, **characterized in that**, for carrying out the second fermentation for preparation of the liquid fermented product 2, a medium comprising of dry pea protein, dry coconut milk, dry coconut flour, pectin, carrageenan, sucrose, dextrose monohydrate and bottled spring water is prepared, which is then sterilized at 98°C for 60 min., cooled down to 37°-45°C, then to the obtained liquid medium the previously thermally treated and cooled down to a temperature of 37°- 45°C cryoprotectant of sugar, dextrose monohydrate and bottled spring water is added in a 1.5: 1.2: 2 ratio and the liquid fermented product 1 is added in a 1:3 ratio, after which the resulting mixture is homogenized and left to ferment for 1 to 3 hours at a temperature of 37°- 45°C until pH of 4.5 to 4.7 is reached.

8. Process of preparation of a fermented dietary product according to claim 7, **characterized in that** the components of the medium for preparation of a liquid fermented product 2 are in quantities as follows (in wt%):
dry pea protein - from 2.64 to 2.72; dry coconut milk - from 2.64 to 2.72; dry coconut flour - from 1.32 to 1.36; pectin - from 0.66 to 0.67; carrageenan - from 0.16 to 0.17; sucrose - from 0.33 to 0.34; dextrose monohydrate - from 0.33 to 0.34 and bottled spring water - from 61.75 to 62.93.

9. Process of preparation of a fermented dietary product according to claims 1-8, **characterized in that** the liquid fermented product 2 is cooled down to a temperature of 20°C and subjected to lyophilization.

10. Fermented dietary product containing:
- dry substance from 12.1 to 13.31 wt % with humidity up to 6% and pH value after rehydration from 5.5 to 6.9;
- live cells of the *Lactobacillus delbrueckii* subsp. *bulgaricus* species in an amount from 2.8 × 10⁸ cfu/g to 5.0 × 10⁸ cfu/g and live cells of the *Streptococcus thermophilus* species in an amount from 1.7 × 10⁷ cfu/g to 5.4 × 10⁷ cfu/g, maintaining their viability for 24 months at a room temperature up to 24°C.

11. Fermented dietary product according to claim 10, **characterized in that** it further includes a dry extract of olive fruit or magnesium oxide or zinc pidolate or inulin.

12. Fermented dietary product according to claims 10-11, for use as a food or food supplement for people following vegetarian, vegan or parve diet as well as to achieve effects on the human health such as improving lactose digestion in individuals with lactose intolerance, protection of lipids in blood from oxidative stress, maintenance of the normal function of the immune system, maintenance of the normal mental and cognitive function, maintenance of the normal condition of hair, nails, bones, teeth and skin, the normal protein and DNA synthesis and the normal carbohydrate metabolism.
